# EUROPEAN PATENT SPECIFICATION

(11) **EP 0 251 512 B1**
(45) Date of publication and mention of the grant of the patent: **31.08.1994**
(21) Application number: 87304967.0
(22) Date of filing: 04.06.1987
(51) Int. Cl.: A61B 17/22, A61M 1/00, A61M 5/142

(54) **Apparatus for removing gallstones**
Gerät zur Beseitigung von Gallensteinen
Appareil pour enlever des calculs biliaires

(30) Priority: 09.06.1986 US 871775
(43) Date of publication of application: 07.01.1988
(73) Proprietor: THE REGENTS OF THE UNIVERSITY OF CALIFORNIA, Oakland, California 94612-3550 (US)
(72) Inventor: Zakko, Salam, San Diego California 92111 (US)
(74) Representative: Kirschner, Klaus Dieter, Dipl.-Phys.

(56) References cited:
- WO-A-87/00759
- AT-B- 297 208
- DE-A- 2 230 283
- DE-A- 2 946 444
- US-A- 3 316 910
- US-A- 3 329 147
- US-A- 3 410 268

## Description

For most of the 500,000 plus individuals who suffer from gallstones, the treatment of choice is to have a cholecystectomy, or removal of the gallbladder. Recently, because of the cost and possible side effects associated with surgery, methods have been developed for chemically removing gallstones in situ. Generally, this procedure requires inserting a catheter into the gallbladder followed by infusing a chemical solvent capable of dissolving the gallstone. The procedure thus avoids the need and attendant risk of surgery.

A variety of chemical solvents have been tried and found to exhibit varying efficiencies of gallstone dissolution, depending on the chemical nature of the gallstone. Gallstones are generally composed of cholesterol, or calcium salts, particularly calcium bilirubinate and calcium carbonate. Lipid solvents are effective at dissolving cholesterol gallstones, whereas these solvents have little or no solubilizing effect on gallstones composed of calcium salts. Thus, diethyl ether readily dissolves cholesterol gallstones, and other solvents such as mono-octinon, and octodiol (glyceryl-l-octyl ether) have good to outstanding solubilizing properties. Unfortunately, few if any solvents are satisfactory for dissolving calcium gallstones.

In addition to the solvents described above suitable for dissolving cholesterol, recently it has been recognized that methyl tert butyl ether (MTBE), a solvent hithertofore used primarily as a gasoline additive and a chromatographic solvent media, was discovered to have outstanding cholesterol gallstone dissolving properties. Moreover, the solvent rapidly dissolves the gallstones without damaging the mucosa of the gallbladder. This finding has led to considerable activity focused on developing apparatus and methods for delivering MTBE to patients suffering from gallstones in ways to most effectively solubilize gallstones without the complications arising from introducing a solvent into the body.

It is obvious that delivering a solvent to a patient requires the utmost care to avoid releasing the solvent into the patient's bodily fluids or outside the area of treatment. Thus a key consideration in developing devices used in the chemical therapy of gallstone dissolution is ensuring the controlled delivery and removal of the solvent used to dissolve the gallstones. Considering that studies have shown that MTBE is injurious if it passes into the intestine where it gets absorbed which may also be an effect of other solvents used to solubilize lipids, there is a critical need for devices that ensure that such chemicals will not be released during chemical therapy for gallstone removal.

Also, because of the need to ensure containment of the effective solvent, in addition to the safety features described above, a suitable device should be "user friendly," and not require the presence of highly skilled technicians to run the device. Further, for the same reasons, it should be easily maintainable.

With a little reflection, it becomes apparent that there are considerable hurdles to surmount if one is to develop a device that has the features described above. For instance, it must be "intelligent" and capable of sensing instantaneous changes in gallbladder pressure brought about by infusing the solvent, and rapidly relay this information to controlling feedback circuits. This is a crucial feature for such a device. If a gallstone should in some way prevent the necessary circulation of the solvent through the gallbladder, a critical pressure will build up, possibly rupturing the organ, or causing leakage of the solvent from the gallbladder through the cystic duct into the common duct and intestine. Thus the device must be "intelligent" in the sense that it senses gallbladder pressure changes over a predefined range, and reacts fast enough to keep the pressure in that range, shutting down when the pressure is outside the range. Moreover, ideally, it would be desirable to have a device that not only is capable of shutting down, but actually can flush out any debris causing the blockage, and resume normal operation should the debris be removed.

In the past there have been a number of proposals for systems for irrigation of certain organs or joints during surgery, but none have dealt with solvent systems for dissolution of internal concretions, specifically gallstones. For instance, Desatnick et al in WO87/00759 (intermediate document) propose an irrigation system for knee surgery which uses a single infusion pump which may be controlled to regulate excess pressure irrigation fluid. A simple drain allows excess fluid to escape from the joint when the infusion pump is slowed or stopped. Similarly, Perez in US 4261360 proposes a system in which excess transurethral pressure occurring during bladder surgery triggers an alarm, alerting medical personnel to slow or stop infusion of irrigation fluid. Additionally, Wallach in

US 4024866 discloses a system for detecting excess intraocular pressure during eye surgery with a pulsating "fluid knife" and regulating the knife's liquid pressure to allow the excess pressure to be reduced. Also to be noted is an article by Allen et al., "Rapid Dissolution of Gallstones by Methyl tert-Butyl Ether," N. Eng. J. Med., 322(4): 217-220 (24 January 1985), which reports two clinical cases which demonstrated that methyl tert-butyl ether administered by percutaneous transhepatic catheter can induce rapid dissolution of cholesterol gallstones in the gallblatter or bile duct without causing side effects in patients. One minute cycles of infusion and complete aspiration were performed to create uninterrupted stirring within the gallbladder.

The present invention proposes a unique system of solvent and specifically designed apparatus which is particularly adapted for use in the therapeutic treatment of obstructions in the gallbladder of a patient caused by cholesterol gallstones. More particularly, the invention provides, in combination, a solvent capable of dissolving gallstones and an apparatus for use therewith, the apparatus comprising pumping means operable to receive the solvent from a reservoir and adapted for pumping the solvent via catheter to a gallbladder for smoothly infusing and aspirating the solvent therethrough; the pumping means comprising an infusion pump and an aspiration pump; with the apparatus further comprising a pressure transducer adapted for sensing a pressure operably reflecting pressure within the gallbladder (intra-gallblader pressure) and a controller responsive to the transducer and operably adapted to control the pumping means within a preset normal operating range of intra-gallbladder pressure to infuse the solvent into the gallbladder and to aspirate from the gallbladder fluid comprising solvent and gallstone material, the controller being operably responsive above the upper limit of the preset normal operating range to cause infusion of fluid to the gallbladder via the infusion pump to be interrupted to enable nominal operating pressure to be re-established, and the controller being operably responsive below the lower limit of the preset normal operating range to cause the aspiration pump to stop aspirating the fluid.

The apparatus is preset to perfuse at a set pressure range. Over this range the solvent is constantly passed from a reservoir into the gallbladder, and from the gallbladder it is aspirated to a suitable receptacle. As shown in the illustrated embodiment, this may be the original reservoir. Delivery and removal of the solvent is at a rate sufficient to effect gallstone dissolution. Should there be an increase in pressure, a feedback loop activates the device into the high pressure mode and infusion of the solvent to the gallbladder via the infusion pump to be interrupted. If after a predetermined period of time the pressure sensing transducer readings from the gallbladder indicate a return to normal operating pressure range, the device automatically reinitiates the normal infusion and aspiration (perfusion) mode. The apparatus described also has a self purging mechanism. After a preset interval, if the pressure does not decrease, the device enters a reverse mode to purge the aspiration port from the gallbladder when fluid is aspirated backward through the infusion port and infused through the aspiration port to purge it for discrete short intervals, during which time the pressure in the organ is continuously monitored. Should the blockage be removable by this "self-purging" action, the pressure transducers indicate normal operating pressure, and the device again resumes action in the normal pressure mode. However, should the obstruction not be removable, then an alarm circuit is activated, so notifying the user. A further feature of the specifically described apparatus is that it is able to distinguish pressure changes occurring within the gallbladder from those arising as a result of coughing, laughing or like behaviour. This feature prevents needless changes of operating modes.

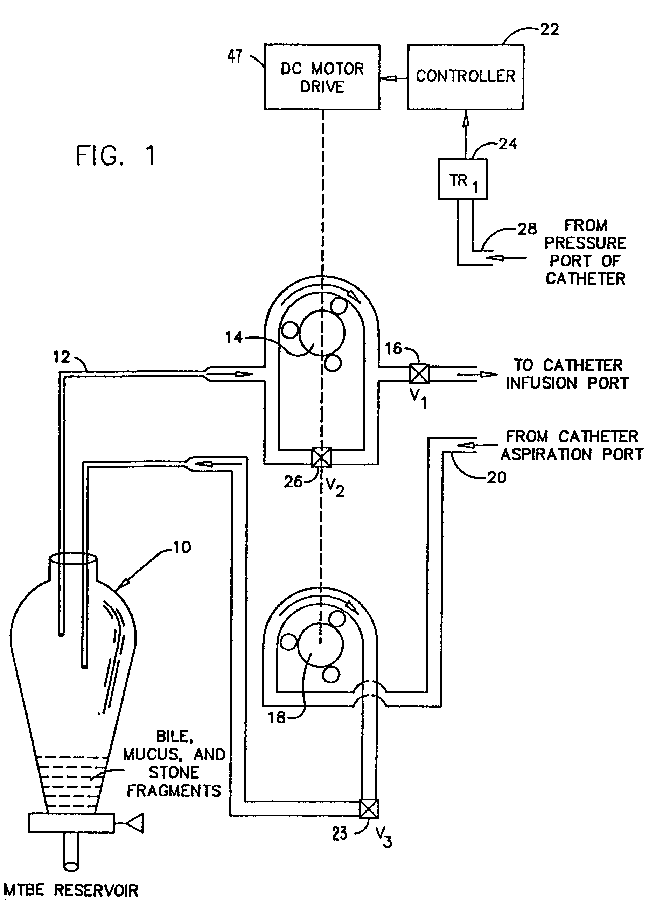

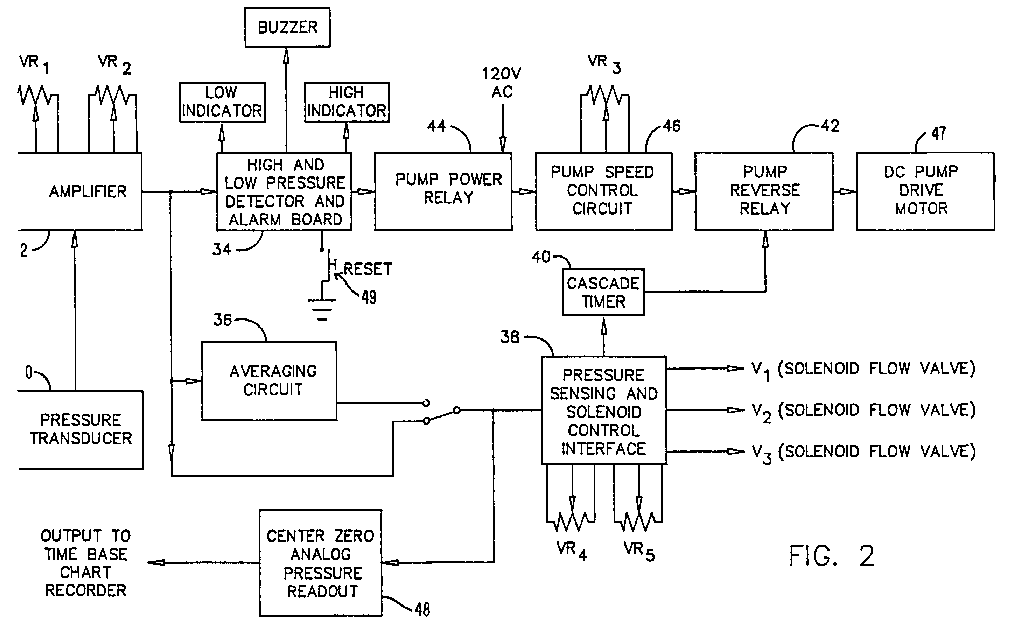

The invention is hereinafter more specifically described by way of example with reference to the accompanying drawings, in which:
Diagram 1 shows an embodiment of apparatus in accordance with this invention suitable for delivering solvent to a gallbladder and for removing the same containing dissolved or fragmented gallbladder stones;
Diagram 2 shows a controller circuit that regulates the pump units shown in Figure 1, as well as other features of the apparatus; and
Diagrams 3, 4 and 5 show features of a 3-lumen catheter.

The apparatus described hereinbelow is specifically adapted for delivering fluids to gallbladders for removing obstructions contained therein.

The apparatus described herein is a gallbladder pressure sensitive apparatus having a solvent delivery means in constant communication with a controller circuit via pressure transducers that monitor intra-gallbladder pressure. The apparatus functions over a preset pressure range delivering fluid to the gallbladder causing the fluid to contact and dissolve the gallstones, and withdrawing fluid from the gallbladder, thereby effecting the removal of dissolved or fragmented gallstones. The rate of solvent delivery and removal can be adjusted to best effect dissolution of the obstruction to create the necessary turbulence to dissolve or fragment gallstones. If the pressure exceeds that of the normal operating range, infusion of solvent to the gallbladder is interrupted, thereby preventing leakage of the solvent from the site of treatment. Further, above the normal operating pressure range, the apparatus can be programmed to be "self-purging". This may be desirable in the instance when the obstruction is only partially dissolvable, causing blockage of the solvent removal, or aspiration means. At lower pressure, or pressure below the normal operating pressure range, the rate of aspiration is decreased while infusion continues, thereby reestablishing normal operating pressure.

Diagram 1 shows an exemplary apparatus for removing gallstones. A reservoir 10 contains a solvent that is a chemical suitable for dissolving gallstones. Should the gallstone be composed of cholesterol, a variety of solvents would be efficacious. Particularly effective is methyl tert butyl ether (MTBE). The latter, as described supra has been shown to readily dissolve cholesterol stones rapidly both in vitro and in vivo. At normal operating pressures, the solvent moves via a conduit 12 from the reservoir by aid of a pumping apparatus 14. The fluid then moves through a valve 16 and from the valve through a catheter into the gallbladder. During this operation valve 26 is closed to prevent solvent return to reservoir. The solvent is delivered at a predetermined effective rate for gallstone dissolution thereby providing solvent turbulance and contact with the gallstones for a period of time sufficient to dissolve and fragment the stones.

Simultaneous with the delivery of MTBE to the gallbladder, a second pump device 18 aspirates the fluid from the gallbladder now containing dissolved gallstones and debris. This material passes out of the gallbladder via an aspiration port in the catheter 20. The fluid moves from the catheter, passing through a valve 23, and from there it is deposited in a reservoir. Either the reservoir 10 used to deliver the solvent, or a separate reservoir is suitable for this purpose. Figure 1 shows the same reservoir 10 being utilized to both deliver fluid to the gallbladder as well as receive aspirated fluid therefrom. It is worth noting that if the same reservoir is used, gallbladder stone fragments, mucous and the like removed from the gallbladder are heavier than the solvent, MTBE, and therefore settle to the bottom of the reservoir and do not hinder continued withdrawal of fluid from the reservoir to effect further stone dissolution.

The pumps 14 and 18 respectively act as infusion pump and aspiration pump and are controlled by a controller circuit 22. The controller circuit 22 in turn receives pressure readings from the transducer 24 causing the controller circuit 22 to open or close flow valves 16, 23 and 26 in a certain fashion to inhibit infusion or aspiration as necessary to control organ pressure depending on whether the transducer 24 indicates that the pressure in the gallbladder is within, above, or below the normal operating pressure range. The transducer in turn is connected to the catheter through port 28 which is in communication with the gallbladder.

At the preset normal operating pressure, the pump 14 delivers fluid from the reservoir 10 to the tube 12 through the valve 16 and to the gallbladder. Simultaneously, and at a slightly slower rate, the pump 18 aspirates the fluid from the gallbladder through the catheter aspiration port 20. Fluid passes through the valve 23 and thence to a reservoir.

The controller is further programmed to respond to pressures that exceed or are below that of the normal operating pressure range. Above the normal operating pressure range (high pressure mode), the controller unit 22 shuts down valve 16 and simultaneously opens valve 26. As a result flow to the gallbladder is interrupted. As the pump 14 continues to operate in this embodiment, fluid is continuously pumped in a closed loop including the now open valve 26. At that time valve 23 is open to effect continual gallbladder emptying to return the pressure to the normal operating range. If the pressure in the gallbladder does not return to the normal operating pressure setting within a preset time, for example, 7 seconds, then the controller can be programmed to instruct the pumps to reverse the direction of fluid movement, and simultaneously valves 23 and 26 are closed. Valve 16 is opened to provide a path for fluid to be reverse aspirated from the gallbladder in this "self-purging" mode. This mode essentially causes a small amount of fluid to be pumped in through the aspiration port of the catheter 20 to clear it from obstructions while aspiration is effected by pump 14 through valve 16. This mode of operation continues for a brief period of time, and then the controller unit 22 instructs the machine to resume normal operation should the obstruction be removed and the pressure transducer 24 indicate reestablishment of normal operating pressure range. If the transducer continues to indicate pressures present in the gallbladder, above the normal operating pressure, the controller unit 22 again instructs the pumping apparatus to purge the system. If, after several "self purging" cycles, the obstruction is still not removed, the controller unit 22 then shuts down the pumping system and activates an alarm circuit 34 notifying the user of a potentially dangerous condition.

Referring now to Diagram 1 and Diagram 2, a representative controller unit will be described. The controller circuit 22 instructs the pumps 14 and 18 to deliver or aspirate fluid from the gallbladder. Thus, a circuit will typically have a pressure transducer 30, and an example of a suitable transducer is a Statham gold pressure transducer P23ID. The pressure transducer 30 relays information to an amplication device 32 which amplifies the signal, and transmits it to a high and low pressure alarm circuit 34, and either directly or through the averaging circuit 36, to a pressure-sensing circuit 38 that reads preset low and high pressure values, and which is connected to the valves 16, 23, and 26. The latter valves are typically solenoid flow valves. The averaging circuit 36 can be switched in if desired to discriminate between pressure changes in the gallbladder arising from fluid buildup due to obstructions, or from hyperventilating, laughing or like activities. Thus the averaging circuit essentially screens out artificially high or low pressure peaks.

The pressure sensing circuit 38 is connected to a cascade timer 40, which in turn is connected to a pump reverse relay circuit 42. Thus, when gallbladder pressure exceeds that of the normal operating pressure range, and the obstruction is not removed within a few seconds, then the cascade timer 40 activates the pump reverse relay 42. The latter circuit is responsible for "self-purging" the system. Should high pressure persist after several brief "self purging" cycles, then the alarm circuit 34 is activated, stopping the pumping system by shutting off its power supply and sounding an alarm notifying the user. Note that at any time during the pump reverse relay cycle, should the pressure return to within the normal pressure range, the apparatus resumes normal operation.

It will be further noted as shown in Diagram 2 that a pump power relay circuit 44 and a pump speed control circuit 46 are also interactive with the whole system. The pump speed control circuit 46 derives power through the pump power relay 44, which in turn is controlled by the alarm circuit 34. The pump motor derives its power supply from the pump power relay 44. Any time an alarm condition exists, this relay shuts off power to the pump, stopping it from pumping. The pump speed control circuit 46 has a manual adjustment capability through which the operator can set the desired perfusion rate for that specific situation. An analogue pressure readout 48 is provided for the operator to assess effective operation and to refer to during calibration.

Several features of an apparatus as described above that enhance its performance will be described. The pumping device preferred is a peristaltic pump. This type of pump offers several advantages such as replacable wettable surfaces, which are particularly advantageous in those instances where the solvent being used to dissolve the obstruction is at all corrosive. Moreover, peristaltic pumps are resistant to clogging, in contrast to standard syringe type pumps. However, it should be noted that syringe pumps are similarly employable in the subject invention in those instances where the fluid used to dissolve and remove the obstruction is a solvent provided the syringe pumps are constructed of suitable material, particularly but not exclusively, glass. Syringe pumps made of plastic are not preferred in instances where the solvents used are incompatible with the plastic composition of the syringe. An additional disadvantage associated with the use of syringe pumps that is not present in peristaltic pumps is that in those instance where a solvent is being utilized, evaporation of the solvent can cause deposits in the body of the syringe, causing it to "freeze," and thus interrupt delivery of the fluid to the organ being treated. Lastly, peristaltic pumps are capable of much greater fluid circulation rates than a syringe pump. This is advantageous in certain instances where the obstruction to be removed requires turbulent flow rates across the surface of the gallstone to accelerate the dissolution process.

A predetermined normal operating pressure range is programmed into the controller circuit 22. Should the pressure in the gallbladder exceed normal operating pressure, the action of the controller circuit 22 prevents leakage of solvent from the gallbladder through the cystic duct into the common duct, as well as into the intestine or around the entry site of the catheter. Also, because the controller circuit "sees" true gallbladder pressure, it readily adjusts to decreased as well as increased pressure by adjusting the net delivery rate of the solvent to the gallbladder. For example, should the pressure fall below the normal operating pressure range, the controller circuit 22 ceases or slows down the rate of aspiration of solvent, and simultaneously continues infusing solvent to reestablish normal operating pressure.

The pressure sensitive alarm circuit 34 is constantly monitoring system operation and gallbladder pressure. If gallbladder pressure cannot be brought to normal operating pressure range by the action of the controller circuit 22 in a specified period of time, it will shut down the pumping system and sound an alarm drawing the attention of the operator. The operator, after correcting the problem, can resume normal operation by activating the reset button 49.

The apparatus is completely automatic, and is operable without any significant operator input. Moreover, it is readily converted to a completely closed circuit system in those instances where the therapeutic fluid is combustible. This feature is required for particularly combustible solvents.

A variety of tubing is suitably used with the pumps of the subject apparatus. However, we have found that tubing composed of Tygon special formulation F-4040A is particularly compatible with solvents such as methyl tert butyl ether. Moreover tubing with a large internal diameter is favoured for use with peristaltic pumps, enabling a high volume per revolution ratio to be obtained, thereby permitting a low revolution per minute rate to be utilized, hence minimizing torque buildup when a switchover to the high pressure mode leads to pump motor reverse.

In using the described apparatus to remove gallstones from gallbladders, perfusion rates of about 50 ml/min - 300 ml/min may be effective. This is readily accomplished by manually adjusting the pump speed control circuit 46.

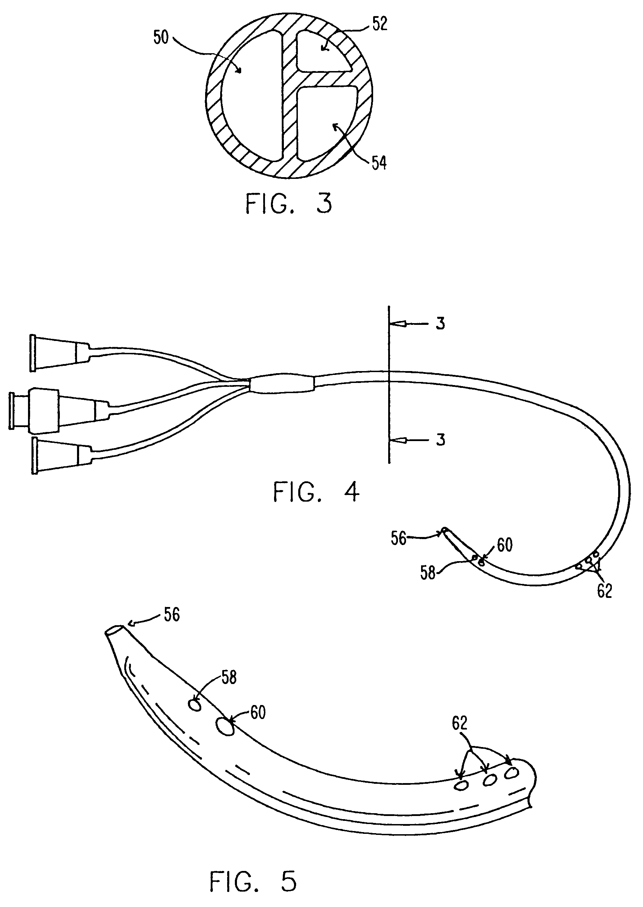

A variety of catheters usable to deliver and aspirate the fluid can be suitably employed. However, a three lumen catheter as shown in Figures 3 and 4 is favoured since pressure measurements, as well as perfusion and aspiration of the fluid can all be carried out simultaneously. A suitable 3-lumen catheter should have an outside diameter not larger than can be readily employed for the surgical insertion of the catheter into the gallbladder, and should have an aspiration port 50, a pressure sensing port 52, and an infusion port 54. To effectively remove dissolved gallstone debris and the like, it is preferred that the aspiration port be larger in diameter than either of the two other ports. Figures 4 and 5 further show another feature of the catheter. It has an elongate tapered tip 56, holes in the tip 58 and 60 that provide a means for fluid communication with the pressure sensing port, and the infusion port, respectively. In addition there are openings 62 in the wall of the catheter that provide a means for fluid communication of fluid in the gallbladder with the aspiration port. Note that the number of openings is not invariant, and depending on the number of gallstones present in the gallbladder, as well as the desirable therapeutic need to effect rapid treatment, that more or fewer holes can be anticipated.

The system should not be construed as being limited to a 3-lumen catheter. A variety of catheters of different lumens will perform satisfactorily provided that the system is modified to accommodate such catheters, such modifications being well known to those skilled in the art.

It will be appreciated by those skilled in the art that there are numerous modifications in both the electrical circuitry, as well as the overall interconnecting features of the apparatus that will achieve the efficacious removal of gallstones from gallbladders. For instance, while the automatic "self purging" feature of the apparatus is desirable, a device without this feature will perform adequately.

## Claims

1. In combination: a solvent capable of dissolving gallstones and an apparatus for use therewith, comprising a pumping means operable to receive from a reservoir (10) said solvent and adapted for pumping the same via catheter means to a gallbladder for smoothly infusing and aspirating the solvent therethrough; the pumping means comprising an infusion pump (14), and an aspiration pump (18); and the apparatus further comprising a pressure transducer (24) adapted for sensing a pressure operably reflecting pressure within the gallbladder (intra-gallbladder pressure), and a controller means (22) responsive to said transducer (24) and operably adapted to control said pumping means within a preset normal operating range of intra-gallbladder pressure to infuse the solvent into the gallbladder and to aspirate from the gallbladder fluid comprising solvent and gallstone material, said controller means (22) being operably responsive above the upper limit of said preset normal operating range to cause infusion of fluid to the gallbladder via said infusion pump (14) to be interrupted to enable nominal operating pressure to be re-established, and said controller means (22) being operably responsive below the lower limit of said preset normal operating range to cause said aspiration pump (18) to stop aspirating said fluid.

2. The combination according to claim 1, further characterized in that said controller means (22) is adapted operably to classify said intra-gallbladder pressure as high, normal or low and to cause said pumping means to maintain normal forward influsion flow at low and normal pressure and to decrease or reverse fluid flow at high pressure.

3. The combination according to claims 1 or 2, further characterized in that said catheter means has three lumens comprising an infusion lumen (54), adapted for normal infusion, an aspiration lumen (50) adapted for normal aspiration, and a pressure-transmitting lumen (52) adapted for operably communicating intra-gallbladder pressure to said pressure transducer.

4. The combination according to any of the foregoing claims, further characterized in that said controller means (22) is adapted operably to respond to pressure from said pressure transducer (24) averaged over time.

5. The combination according to any of the foregoing claims, further characterized in that said pump means is adapted operably to infuse the solvent into the gallbladder at a rate in the range between about 50 ml/min and 300 ml/min.

6. The combination according to any of the foregoing claims, further characterized in that said apparatus is programmed operably to respond to occurrence of a selected irregular pressure condition in said gallbladder by automatically momentarily reversing flow to clear a possible obstruction to normal flow.

7. The combination according to any preceding claim, further characterized in that fluid conduit means (12, 20) are attached to said pump means (14, 18) and form therewith a fluid circuit with said reservoir (10) and said catheter means.

8. The combination according to claim 7, further characterized in that said fluid conduit means comprises first fluid conduit (12) coupling said pump means (14) with said catheter means for operable infusion into said gallbladder, a bypass conduit connected to said first conduit and bypassing said infusion pump, and a second fluid conduit (20) containing said aspiration pump (18) and coupled to said catheter means for operably receiving fluid aspirated from said gallbladder, and in that said controller means (22) is operably adapted to classify intra-gallbladder pressure as high, normal or low, and includes a first valve (16), situated in said fluid conduit and operably adapted to be open at low or normal pressures and closed at high pressure, a second valve situated in said bypass conduit (26) and operably adapted to be closed at low or normal pressures and open at high pressure, and a third valve (23) situated in said second fluid conduit (20) and operably adapted to be open at high or normal pressures and closed shortly after detection of low pressure.

9. The combination according to claim 7, further characterized in that said fluid conduit means comprises first and second conduits (12, 20) adapted respectively for flow of fluid into the infusion direction from said reservoir (10) into the gallbladder, and for flow of fluid in the aspiration direction from the gallbladder to a reservoir (10); and in that said infusion pump (14) is operably associated with said first conduit (12) and said aspiration pump (18) is operably associated with said second conduit (20) whereby said pumping means enables simultaneous infusion and aspiration, with continuous withdrawal of fluid after exposure to gallstones at a substantial rate over a prolonged period.

## Patentansprüche

1. In Kombination: ein Lösungsmittel, das Gallensteine auflösen kann und ein Gerät zur Verwendung mit diesem, mit einer Pumpeinrichtung, die so bedienbar ist, daß sie von einem Speicher (10) das Lösungsmittel erhält, und geeignet ist, dieses über eine Kathetereinrichtung zu einer Gallenblase zu pumpen, um das Lösungsmittel ruhig durch diese zu infundieren und abzusaugen; die Pumeinrichtung weist eine Infusionspumpe (14) und eine Absaugpumpe (18) auf, und das Gerät umfaßt außerdem einen Druckgeber (24), der betriebsgemäß geeignet ist, einen Rückstau-Druck in der Gallenblase (Intra-Gallenblasen-Druck) wahrzunehmen, und eine Steuereinrichtung (22), die auf den Druckgeber (24) reagiert und betriebsfähig geeignet ist, die Pumpeinrichtung innerhalb eines vorgegebenen normalen Betriebsbereiches des Intra-Gallenblasendrucks zu steuern, um das Lösungsmittel in die Gallenblase zu infundieren und Lösungsmittel und Gallenstein-Material enthaltendes Fluid aus der Gallenblase abzusaugen, wobei die Steuereinrichtung (22) über der oberen Grenze des vorgegebenen normalen Betriebsbereiches betriebsgemäß so reagiert, daß sie die Unterbrechung der Infusion des Fluids in die Gallenblase über die Infusionspumpe (14) bewirkt, um zu ermöglichen, daß der Nenn-Betriebsdruck wiederhergestellt werden kann, und wobei die Steuereinrichtung (22) unter der unteren Grenze des vorgegebenen normalen Betriebsbereiches betriebsgemäß so reagiert, daß sie bewirkt, daß die Absaugpumpe (18) das Absaugen des Fluids beendet.

2. Kombination nach Anspruch 1, **weiter dadurch gekennzeichnet, daß** die Steuereinrichtung (22) betriebsgemäß geeignet ist, den Intra-Gallenblasendruck als hoch, normal oder niedrig zu klassieren und die Pumpeinrichtung zu veranlassen, den normalen Vorwärts-Infusionsfluß bei niedrigem und normalem Druck aufrechtzuerhalten und den Fluidfluß bei hohem Druck zu reduzieren oder umzukehren.

3. Kombination nach Anspruch 1 oder 2, **weiter dadurch gekennzeichnet, daß** die Kathetereinrichtung drei Lumen aufweist, umfaßend einen für normale Infusion geeigneten Infusionslumen (54), einen für normales Absaugen geeigneten Absauglumen (50), und einen Druckübertragungslumen (52), der geeignet ist, den Intra-Gallenblasendruck an den Druckgeber betriebsgemäß zu übertragen.

4. Kombination nach einem der vorhergehenden Ansprüche, **weiter dadurch gekennzeichnet, daß** die Steuereinrichtung (22) betriebsgemäß geeignet ist, auf den über die Zeit auf einen Mittelwert gebrachten Druck des Druckgebers (24) zu reagieren.

5. Kombination nach einem der vorhergehenden Ansprüche, **weiter dadurch gekennzeichnet, daß** die Pumpeinrichtung betriebsgemäß geeignet ist, das Lösungsmittel in die Gallenblase mit einer Rate im Bereich zwischen ca. 50 ml/min und 300 ml/min zu infundieren.

6. Kombination nach einem der vorhergehenden Ansprüche, **weiter dadurch gekennzeichnet, daß** das Gerät so programmiert ist, das es betriebsgemäß auf das Auftreten einer gewählten unregelmäßigen Druckbedingung in der Gallenblase so reagiert, daß es automatisch den Fluß momentan umkehrt, um ein mögliches Hindernis zu entfernen, zum normalen Fluß.

7. Kombination nach einem der vorhergehenden Ansprüche, **weiter dadurch gekennzeichnet, daß** eine Fluid-Kanal-Einrichtung (12,20) mit der Pumpeinrichtung (14,18) verbunden ist und mit dieser einen Fluidkreislauf mit dem Speicher (10) und der Kathetereinrichtung bildet.

8. Kombination nach Anspruch 7, **weiter dadurch gekennzeichnet, daß** die Fluid-Kanaleinrichtung einen ersten Fluidkanal (12) aufweist, der die Pumpeinrichtung (14) mit der Kathetereinrichtung für eine betriebsgemäße Infusion in die Gallenblase koppelt, wobei ein Seitenkanal mit dem ersten Kanal verbunden ist und die Infusionspumpe umgeht, und einen zweiten Fluidkanal (20), der die Absaugpumpe (18) enthält und mit der Kathetereinrichtung gekoppelt ist, um betriebsgemäß von der Gallenblase abgesaugtes Fluid zu erhalten; und daß die Steuereinrichtung (22) betriebsgemäß geeignet ist, den Intra-Gallenblasendruck als hoch, normal oder niedrig zu klassieren, und enthält:
ein in dem Fluidkanal angeordnetes erstes Ventil (16), das betriebsgemäß geeignet ist, bei niedrigem oder normalem Druck offen und bei hohem Druck geschlossen zu sein,
ein in dem Nebenkanal (26) angeordnetes zweites Ventil, das betriebsgemäß geeignet ist, bei niedrigem oder normalem Druck geschlossen zu sein und bei hohem Druck offen zu sein,
und ein in dem zweiten Fluidkanal (20) angeordnetes drittes Ventil (23), das betriebsgemäß geeignet ist, bei hohem oder normalem Druck offen und kurz nach dem Erfassen von niedrigem Druck geschlossen ist.

9. Kombination nach Anspruch 7, **weiter dadurch gekennzeichnet, daß** die Fluidkanaleinrichtung einen ersten und einen zweiten Kanal (12,20) aufweist, die jeweils für den Fluß von Fluid in die Infusionsrichtung vom Speicher (10) in die Gallenblase und für den Fluß von Fluid in die Absaugrichtung von der Gallenblase zu einem Speicher (10) geeignet sind; und daß die Infusionspumpe (14) betriebsgemäß mit dem ersten Kanal (12) verbunden ist, und die Absaugpumpe (18) betriebsgemäß mit dem zweiten Kanal (20) verbunden ist, wobei die Pumpeinrichtung eine gleichzeitige Infusion und Absaugung mit kontinuierlichem Rückzug von Fluid, nachdem es den Gallensteinen ausgesetzt wurde, bei einer dauerhaften Rate über einen langen Zeitraum ermöglicht.

## Revendications

1. En association : un solvant capable de dissoudre les calculs biliaires et un appareil à utiliser avec ce dernier, comprenant un moyen de pompage fonctionnant pour recueillir ledit solvant d'un réservoir (10) et conçu pour l'envoyer par pompage, par l'intermédiaire de moyens formant cathéter, à une vésicule biliaire pour y perfuser et en aspirer avec douceur le solvant, le moyen de pompage comprenant une pompe à perfusion (14) et une pompe d'aspiration (18), et l'appareil comprenant en outre un transducteur de pression (24) conçu pour détecter une pression réfléchissant de façon fonctionnelle la pression à l'intérieur de la vésicule biliaire (pression intravésiculaire) et un moyen de commande (22) réagissant audit transducteur (24) et conçu fonctionnellement pour commander audit moyen de pompage au sein d'une plage de fonctionnement normal prédéterminée de pressions intravésiculaires de perfuser le solvant dans la vésicule biliaire et aspirer de la vésicule biliaire le liquide comprenant le solvant et les calculs, ledit moyen de commande (22) réagissant fonctionnellement au-delà de la limite supérieure de ladite plage de fonctionnement normal prédéterminée de façon à interrompre la perfusion de liquide vers la vésicule biliaire par l'intermédiaire de ladite pompe à perfusion (14) pour permettre le rétablissement de la pression nominale de fonctionnement, et ledit moyen de commande (22) réagissant fonctionnellement en dessous de la limite inférieure de ladite plage de fonctionnement normal prédéterminée pour arrêter ladite pompe d'aspiration (18) aspirant ledit liquide.

2. Association selon la revendication 1, caractérisé en outre en ce que ledit moyen de commande (22) est conçu fonctionnellement pour classer ladite pression intravésiculaire en pression élevée, normale ou faible et pour amener ledit moyen de pompage à maintenir un courant de perfusion normal vers l'avant à des pressions faibles et normales et à diminuer ou inverser le courant de liquide à une pression élevée.

3. Association selon les revendications 1 ou 2, caractérisée en outre en ce que ledit moyen formant cathéter présente trois lumières comprenant une lumière de perfusion (54), conçue pour une perfusion normale, une lumière d'aspiration (50), conçue pour une aspiration normale, et une lumière de transmission de la pression (52), conçue pour communiquer fonctionnellement la pression intravésiculaire audit transducteur de pression.

4. Association selon l'une quelconque des revendications précédentes, caractérisée en outre en ce que ledit moyen de commande (22) est conçu fonctionnellement pour réagir à la pression fournie par ledit transducteur de pression (24), dont la moyenne est établie au fil du temps.

5. Association selon l'une quelconque des revendications précédentes, caractérisée en outre en ce que ledit moyen de pompage est conçu fonctionnellement pour perfuser le solvant dans la vésicule biliaire à un débit compris dans la plage d'environ 50 ml/min à 300 ml/min.

6. Association selon l'une quelconque des revendications précédentes, caractérisée en outre en ce que ledit appareil est programmé fonctionnellement pour réagir à la survenue d'un état de pression irrégulière sélectionné, dans ladite vésicule biliaire, par une inversion automatique temporaire du courant pour éliminer une possible obstruction au courant normal.

7. Association selon l'une quelconque des revendications précédentes, caractérisée en outre en ce que des moyens formant des conduits de liquide (12, 20) sont fixés audit moyen de pompage (14, 18) et forment avec ce dernier un circuit de liquide avec ledit réservoir (10) et ledit moyen formant cathéter.

8. Association selon la revendication 7, caractérisée en outre en ce que ledit moyen formant des conduits de liquide comprend un premier conduit de liquide (12) reliant ledit moyen de pompage (14) audit moyen formant cathéter en vue de la perfusion fonctionnelle de ladite vésicule biliaire, un conduit de pontage relié audit premier conduit et court-circuitant ladite pompe à perfusion, et un second conduit de liquide (20) contenant ladite pompe d'aspiration (18) et couplé audit moyen formant cathéter pour recueillir fonctionnellement le liquide aspiré de ladite vésicule biliaire; et en ce que ledit moyen de commande (22) est conçu fonctionnellement pour classer ladite pression intravésiculaire en pression élevée, normale ou faible et comprend une première soupape (16), située dans ledit conduit de liquide et conçue fonctionnellement pour être ouverte a des pressions basses ou normales et fermée à une pression élevée, une seconde soupape située dans ledit conduit de pontage (26) et conçue fonctionnellement pour être fermée à des pressions basses ou normales et ouverte à une pression élevée, et une troisième soupape (23) située dans ledit second conduit de liquide (20) et conçue fonctionnellement pour être ouverte à des pressions élevées ou normales et fermée brièvement après la détection d'une pression basse.

9. Association selon la revendication 7, caractérisée en outre en ce que ledit moyen formant des conduits de liquide comprend un premier et un second conduit (12,20) conçus respectivement pour le courant de liquide dans la direction de perfusion, dudit réservoir (10) à la vésicule biliaire, et pour le courant de liquide dans la direction d'aspiration, de la vésicule biliaire à un réservoir (10); et en ce que ladite pompe à perfusion (14) est fonctionnellement associée audit premier conduit (12) et que ladite pompe d'aspiration (18) est fonctionnellement associée audit second conduit (20), ledit moyen de pompage permettant une perfusion et une aspiration simultanées, pour un retrait continu du liquide après son exposition à des calculs biliaires à une vitesse sensible pendant une durée prolongée.
